(19) Europäisches Patentamt
European Patent Office
Office européen des brevets

(11) Veröffentlichungsnummer: **0 482 477 B1**

# (12) EUROPÄISCHE PATENTSCHRIFT

(45) Veröffentlichungstag der Patentschrift: **11.01.95**

(51) Int. Cl.[6]: **C07D 251/16**, C07D 251/22

(21) Anmeldenummer: **91117555.2**

(22) Anmeldetag: **15.10.91**

## (54) Verfahren zur Herstellung von 6-Trifluormethyl-1,3,5-triazinen.

(30) Priorität: **26.10.90 DE 4034078**

(43) Veröffentlichungstag der Anmeldung:
**29.04.92 Patentblatt 92/18**

(45) Bekanntmachung des Hinweises auf die Patenterteilung:
**11.01.95 Patentblatt 95/02**

(84) Benannte Vertragsstaaten:
**AT BE CH DE ES FR GB IT LI NL**

(56) Entgegenhaltungen:
**GB-A- 1 017 450**
**US-A- 3 270 018**

**CHEMICAL ABSTRACTS, vol. 87, 1977, Columbus, Ohio, US; abstract no. 85055R, TSUNODA, MASARU ET AL.: '2-Substituted 4-trichlo romethyl-6-trifluoromethyl-s-triazines.' Seite 605 ; & JP 77 25,785**

**CHEMICAL ABSTRACTS, vol. 88, 1978, Columbus, Ohio, US; abstract no. 37841T, KADOTA, MASARU ET AL.: '2-Substituted-4-trichlor omethyl-6-trifluoromethyl-s-triazines.' Seite 508 ; & JP 77 83,577**

(73) Patentinhaber: **BASF Aktiengesellschaft**
**Carl-Bosch-Strasse 38**
**D-67063 Ludwigshafen (DE)**

(72) Erfinder: **Mayer, Horst, Dr.**
**Faselwiese 19**
**W-6700 Ludwigshafen (DE)**
Erfinder: **Hamprecht, Gerhard, Dr.**
**Rote-Turm-Strasse 28**
**W-6940 Weinheim (DE)**

EP 0 482 477 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, vol. 83, 1975, Columbus, Ohio, US; abstract no. 164133K, TSUJIKAWA, TERUAKI ET AL.: 'Heteocyclic compounds. I. Syntheses of 1,3,5-triazine derivatives and their pharmacological activities.' Seite 550 ; & Yakugaku Zasshi 1975, 95(5), 499-511.

CHEMICAL ABSTRACTS, vol. 87, 1977, Columbus, Ohio, US; abstract no. 53396M, TSUNODA, MASARU ET AL.: '2,4-Disubstituted 6-trifluoromethyl-s-triazines.' & JP 77 25,786

CHEMICAL ABSTRACTS, vol. 88, 1978, Columbus, Ohio, US; abstract no. 89718C, KADOTA, MASARU ET AL.: '2,4-Disubstituted -6-trifluoromethyl-s-triazines.' Seite 527 ; & JP 77 83,582

Journal of the American Chemical Society, vol.79, pages 2629-33

Canadian Journal of Chemistry, vol.35, pages 1285-92(1957)

Chemistry and Industry, 1974, pages 874-5

Chemical Pharm. Bulletin, vol.16, pages 474-9(1968)

Journal of Heterocyclic Chemistry, vol.22, pages 1621-30(1985)

Journal of Organic Chemistry, vol. 39, pages 1836-8(1974)

Journal of Organic Chemistry, vol.44, pages 2906(1979)

## Beschreibung

Die vorliegende Erfindung betrifft ein neues Verfahren zur Herstellung von 6-Trifluormethyl-1,3,5-triazinen

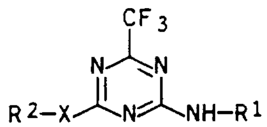

I

der allgemeinen Formel I in der X Sauerstoff , $R^1$, $R^2$ C-organische Substituenten mit 1 bis 6 C-Atomen und $R^1$ zusätzlich Wasserstoff bedeuten.

Aus JP-A-52 025786, JP-A-52 025785 und JP-A-52 083577 ist die Überführung von N-Trichloracetamidino-trichloracetamidin in 2,4-Bis-trichlormethyl-6-trifluormethyl-1,3,5-triazin mit 2,2 Moläquivalenten Trifluoressigsäureanhydrid gemäß folgendem Reaktionsschema

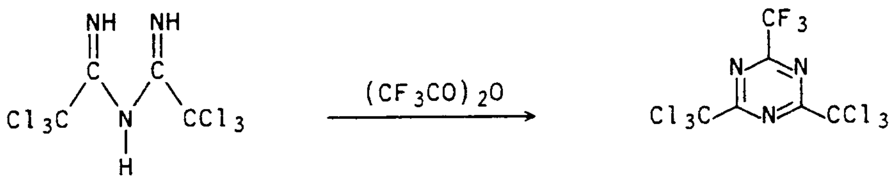


bekannt.

Der formale Ersatz einer der beiden elektronenziehenden Trichlormethylgruppen im N-Trichloracetamidino-trichloracetamidin durch eine elektronendrückende Aminogruppe führt zu den Trichloracetamidinoguanidinen II. Bei ihnen sollte demnach ein Angriff von starken Elektrophilen wie Trifluoressigsäureanhydrid oder -chlorid leichter möglich sein wie bei den elektronenärmeren Amidinen.

In Z. anorg. allg. Chem. 589 (1990) 69-78 wird gelehrt, daß die Umsetzung von N-(Trichlormethylcarbimidoyl)guanidin mit Essigsäureanhydrid zu heteroaromatischen 1,3,5-Triazinsystemen führt. Dagegen gaben Acetylierungsversuche mit Essigsäureethylester unter breiter Variation der Versuchsbedingungen keinen Hinweis auf eine chemische Umsetzung.

Gemäß der Lehre der Can. J. Chem. 35, 1285 (1957) werden Guanidine durch Umsetzung mit Acetanhydrid oder insbesondere Trifluoracetanhydrid in diacylierte Derivate überführt, die in bestimmten Fällen zu Triazinen cyclisiert werden können:

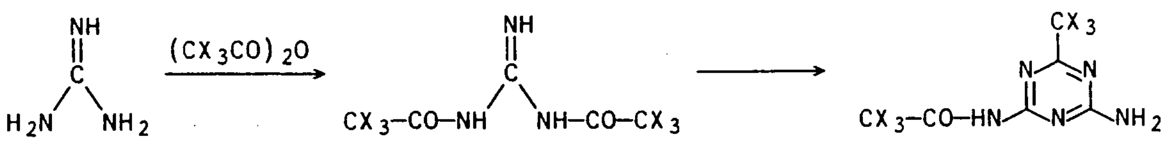


X = H, F.

Der abschließende Cyclisierungsschritt ist jedoch weder mit dem Hydrochlorid des Guanidins noch dem des Cyclohexylguanidins möglich, sobald als Elektrophil Trifluoressigsäureanhydrid verwendet wird, da in diesen Fällen lediglich die Diacylguanidine (X = F) als Reaktionsprodukte erhalten werden.

Auch bei der Umsetzung der N-Trichloracetamidinoguanidine II mit Trifluoressigsäureanhydrid oder -chlorid wäre demnach eine Bisacylierung des Guanidinteils ohne anschließende Cyclisierung der diacylierten Produkte zu erwarten:

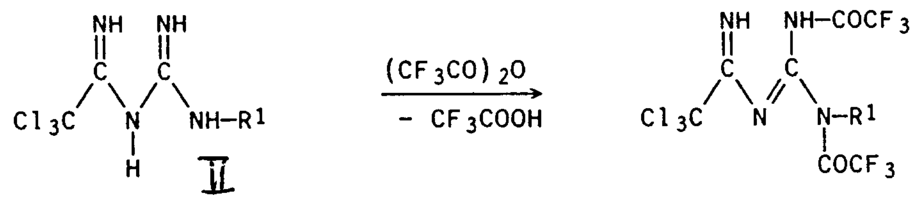


II

Wie in den Druckschriften JP-A 51 068 581, JP-A 52 025 786, JP-A 52 083 576 und JP-A 52 083 582 offenbart wurde, können die 2-Amino-4-trichlormethyl-6-trifluormethyl-1,3,5-triazine IV aber aus den o.g. 2,4-Bis-trichlormethyltriazinen mittels Substitution einer Trichlormethylgruppe durch einen Aminrest hergestellt werden. Technisch unbefriedigend an diesem 2-stufigen Verfahren ist jedoch die Bildung von Chloroform in äquimolaren Mengen als Nebenprodukt. Ferner wäre eine einfachere Synthese mit nur einem Verfahrensschritt wünschenswert.

Bei der vergleichbaren Substitution der verbliebenen Trichlormethylgruppe an der Verbindung IV durch Alkoholate ist als Konkurrenzreaktion die Substitution der drei Fluoratome unter Bildung eines Orthoesters zu erwarten (Williamson-Reaktion; vgl. R.H. de Wolfe in "Carboxylic Ortho Acid Derivatives", Acad. Press., New York, 1970, Seiten 12 bis 18). Insbesondere ist aus J. Heterocycl. Chem. 22, 1621 (1985) bekannt, die Trifluormethylgruppe an einem Thiazol-Grundkörper bereits unter milden Reaktionsbedingungen in einen Orthoester zu überführen:

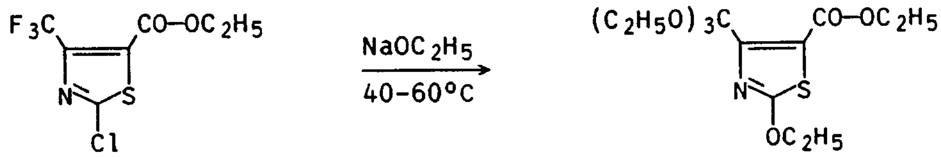


Eine andere Möglichkeit zur Herstellung von 6-Trifluormethyl-1,3,5-triazinen besteht nach einem aus Yakugaku Zasshi 95, 499-511 (1975) bekannten Verfahren darin, N-Cyanoguanidine in Kupferkomplexe von N-Amidino-O-alkylisoharnstoffen zu überführen, die Harnstoffderivate mit Schwefelwasserstoff freizusetzen und anschließend mit Trifluoressigsäureester gemäß folgendem Reaktionsschema

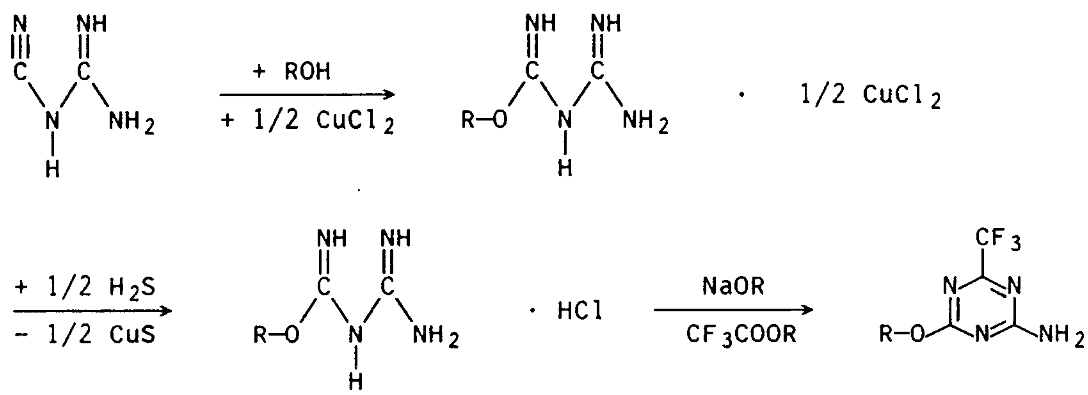


umzusetzen. Aus der DD-A-252 374 ist eine Variante dieses Verfahrens bekannt, wobei anstelle des Kupfer-(II)chlorides das Kupfer(II)acetat verwendet wird, und der im 1. Verfahrensschritt gebildete Kupferkomplex des N-Amidino-O-alkylisoharnstoffs direkt mit Trifluoressigsäureanhydrid zu einem 6-Trifluormethyl-1,3,5-triazin umgesetzt wird.

Ein großer Nachteil der beiden Verfahren besteht jedoch darin, daß bei der Herstellung der N-Amidino-O-alkylisoharnstoffe große Mengen an Kupfersalzen als Nebenprodukt abgetrennt und entsorgt werden müssen, was eine Durchführung im technischen Maßstab unrentabel erscheinen läßt.

Weitere Synthesen von 1,3,5-Triazinen, die von den N-Amidino-O-alkylisoharnstoffen ausgehen, sind z.B. aus DE-A 1 220 431, DE-A 3 324 800, EP-A 98 569, FR-A 11 380 818, US-A 3 258 462, Chem. Pharm. Bull. 16 (3), 474 (1968) und dto., 21 (3), 478 (1973) bekannt. Nachteilig bei diesen Reaktionen ist jedoch die

aus Chem. Pharm. Bull 16 (3), 474 (1968) bekannte Empfindlichkeit von N-Amidino-O-alkylisoharnstoffen gegen Basen wie Natriumethanolat, wobei Cyanoguanidine gebildet werden:

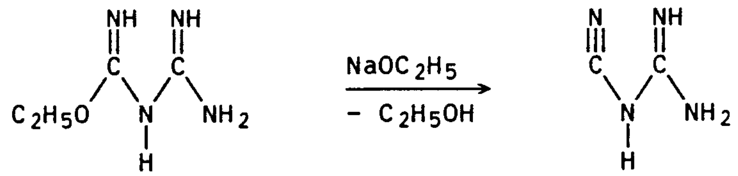


Da die Trichlormethylgruppe der N-Trichloracetamidinoguanidine II ebenfalls eine gute Abgangsgruppe wie die Alkoxygruppe der N-Amidino-O-alkylisoharnstoffeist, sollte die Empfindlichkeit der Verbindungen II gegen Basen vergleichbar sein:

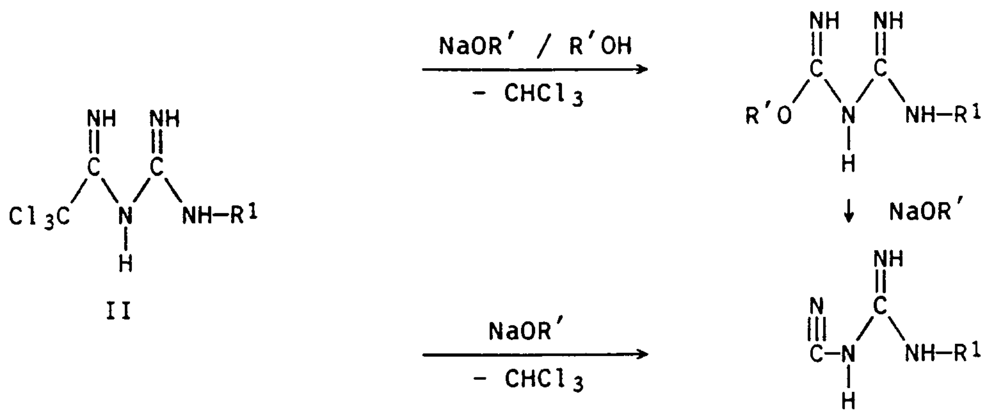


R' = niedere Alkylgruppe

Zur Vermeidung solcher Zersetzungsreaktionen wird in der genannten Literatur entweder ohne Base oder - zur Freisetzung der N-Amidino-O-alkylisoharnstoffe aus ihren Salzen - mit äquimolaren Mengen an Base oder mit einem geringen Überschuß bis etwa 10 mol-%, gearbeitet. Insbesondere bei Anwesenheit von größeren Mengen an Base werden die gewünschten Triazine trotzdem nur in sehr unbefriedigenden Ausbeuten erhalten.

In Journal of the American Chemical Society, Band 79, S. 2629-33 (1957) wird der Austausch einer $CCl_3$-Gruppe am Triazin durch eine Alkoxygruppe gelehrt. Ferner wird gelehrt, daß Ausgangsprodukte mit labilen Substituenten insbesondere in protischen Lösungsmitteln zu Nebenreaktionen neigen.

Der Erfindung lag nun die Aufgabe zugrunde, die Verbindungen I besser zugänglich zu machen.

Demgemäß wurde ein Verfahren zur Herstellung von 6-Trifluormethyl-1,3,5-triazinen der Formel I gefunden, welches dadurch gekennzeichnet ist, daß man

(a) ein N-Trichloracetamidinoguanidin der allgemeinen Formel II

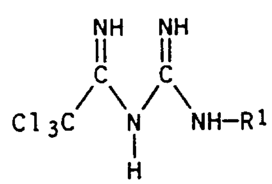
II

mit einem Trifluoressigsäurederivat der allgemeinen Formel III

$CF_3$-CO-Y III

in der Y Chlor, $C_1$-$C_4$-Alkoxy oder Trifluoracetyloxy bedeutet, in Gegenwart oder Abwesenheit einer Base zu einem 4-Trichlormethyl-6-trifluormethyl-1,3,5-triazin der Formel IV

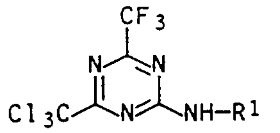

IV

umsetzt und

(b) das Verfahrensprodukt IV in Gegenwart einer Base mit einem Alkohol der allgemeinen Formel V

$R^2$-OH V,

in der $R^2$ einen C-organischen Rest mit 1 bis 6 C-Atomen darstellt, reagieren läßt.

Die als Ausgangsstoffe dienenden N-Trichloracetamidinoguanidine II sind aus Rec. Trav. chim. 70, 638 (1951) [CA 1952, 8610d] bekannt oder lassen sich nach der dort beschriebenen Methode aus Trichloracetonitril und einem N-substituierten Guanidin gemäß dem allgemeinen Reaktionsschema

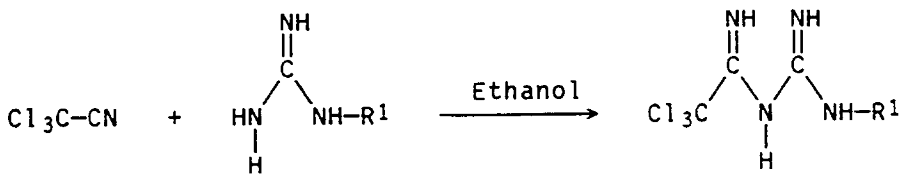


II

herstellen.

Das Guanidin kann auch in Form eines Säureadditionssalzes eingesetzt werden, wobei in diesem Fall die während der Reaktion frei werdende Säure zweckmäßig durch Zugabe einer geeigneten Base wie Natriumethanolat neutralisiert wird.

Als C-organische Reste in Formel I sind für $R^1$ insbesondere Wasserstoff, aliphatische, cycloaliphatische oder aromatische Reste zu nennen, z.B. niedermolekulare Alkyl-, Alkenyl-, Alkinyl- oder Cycloalkylreste. $R^2$ kann z.B. ein aliphatischer oder cycloaliphatischer Rest wie niedermolekularer Alkyl-, Alkenyl- oder Alkinylrest sein.

Im Hinblick auf die Verwendung der herzustellenden Zwischenprodukte I geht man vorzugsweise von solchen Verbindungen II aus, bei denen $R^1$ Wasserstoff, eine $C_1$-$C_4$-Alkylgruppe wie Methyl, Ethyl, n-Propyl, Isopropyl, n-Butyl, sec.-Butyl und tert.-Butyl, eine $C_3$-$C_6$-Cycloalkylgruppe wie Cyclopropyl, Cyclopentyl und Cyclohexyl oder die Phenylgruppe bedeutet. Besonders bevorzugte Reste sind Wasserstoff oder eine $C_1$-$C_4$-Alkylgruppe.

Der Rest $R^2$ in den erfindungsgemäß herzustellenden Verbindungen ist vorzugsweise

- eine $C_1$-$C_4$-Alkylgruppe wie Methyl, Ethyl, n-Propyl, Isopropyl, n-Butyl, sec.-Butyl und tert.-Butyl;
- eine $C_3$-$C_4$-Alkenylgruppe wie Prop-2-en-1-yl, 1-Methyl-prop-2-en-1-yl, But-2-en-1-yl und But-3-en-1-yl;
- eine $C_3$-$C_4$-Alkinylgruppe wie Prop-2-in-1-yl und But-2-in-1-yl;
- eine $C_3$-$C_6$-Cycloalkylgruppe wie Cyclopropyl, Cyclobutyl, Cyclopentyl und Cyclohexyl, bevorzugt Cyclopentyl und Cyclohexyl.

Der Rest $R^2$ kann seinerseits noch weitere (für den Verfahrensschritt (b) inerte) Substituenten tragen.

Als Trifluoressigsäurederivat III eignen sich vornehmlich Trifluoressigsäureanhydrid (Y = Trifluoracetyloxy) und die Ester der Trifluoressigsäure (Y = $C_1$-$C_4$-Alkoxy), wobei der Methyl- und der Ethylester der Trifluoressigsäure ganz besonders bevorzugt sind.

Für die Herstellung der 4-Trichlormethyl-6-trifluormethyl-1,3,5-triazine IV empfehlen sich äquimolare Mengen an N-Trichloracetamidinoguanidin II und Trifluoressigsäurederivat III; bevorzugt werden Mengen von 100 bis 500 mol-%, insbesondere von 200 bis 250 mol-%, an Trifluoressigsäurederivat III, bezogen auf II.

Vorzugsweise arbeitet man in einem inerten Lösungs- bzw. Verdünnungsmittel bei Temperaturen zwischen (-40)°C und der Siedetemperatur des Lösungsmittels, bevorzugt zwischen (-20) und 120°C, insbesondere zwischen (-10) und 100°C.

Als Lösungs- bzw. Verdünnungsmittel eignen sich ganz allgemein Kohlenwasserstoffe wie Hexan, Heptan, Pinan, Octan, Nonan, o-, m-, p-Cymol, Benzinfraktionen mit einem Siedebereich zwischen 70 und

190 °C, Cyclohexan, Methylcyclohexan, Petrolether, Dekalin, Ligroin, 2,2,3-Trimethylpentan, 2,2,4-Trimethylpentan und 2,3,3-Trimethylpentan, Halogenkohlenwasserstoffe wie Dichlormethan, Chloroform, Tetrachlorkohlenstoff, 1,1-Dichlorethan, 1,2-Dichlorethan, 1,1,1-Trichlorethan, 1,1,2-Trichlorethan, 1,1,2,2-Tetrachlorethan, 1,1,1,2-Tetrachlorethan, Pentachlorethan, 1,2-cis-Dichlorethylen, Trichlorethylen, Tetrachlorethylen, Dichlorpropan, Dichlorbutan, Fluorbenzol, Chlorbenzol, Brombenzol, Iodbenzol, o-, m-, p-Difluorbenzol, o-, m-, p-Dichlorbenzol, o-, m-, p-Dibrombenzol, 1,2,4-Trichlorbenzol, o-, m-, p-Chlortoluol, Chlornaphthalin und 1,2-Dichlornaphthalin, Ether wie Diethylether, Ethyl-n-propylether, Diisopropylether, Di-n-butylether, Diisobutylether, n-Butylethylether, Methyl-tert.-butylether, Diisoamylether, Cyclohexylmethylether, Ethylenglykoldimethylether, Anisol, Phenetol, Tetrahydrofuran, Dioxan und $\beta,\beta'$-Dichlordiethylether, Thioether wie Thioanisol, Nitrokohlenwasserstoffe wie Nitromethan, Nitroethan, Nitrobenzol, o-Nitrotoluol und o-, m-, p-Chlornitrobenzol, Nitrile wie Acetonitril, Butyronitril, Isobutyronitril, Benzonitril und m-Chlorbenzonitril, Ester wie Ethylacetat, Acetessigester und Isobutylacetat, Amide wie Formamid, Methylformamid, Dimethylformamid und Diethylformamid, Ketone wie Aceton und Methylethylketon sowie Gemische der genannten Solventien.

Führt man den Verfahrensschritt (a) mit Trifluoressigsäureanhydrid oder Trifluoressigsäurechlorid durch, so ist Diethylether als Lösungsmittel besonders bevorzugt.

Bei Reaktionsführung mit einem Trifluoressigsäureester verwendet man besonders bevorzugt Tetrahydrofuran oder den Trifluoressigester selbst.

Die Menge an Lösungsmittel ist nicht kritisch. Normalerweise verwendet man die 1- bis 5-fache Menge an Lösungsmittel, bezogen auf die Menge an N-Trichloracetamidinoguanidin II.

Besondere Bedingungen bezüglich des Druckes sind nicht erforderlich, im allgemeinen nimmt man die Umsetzung bei Atmosphärendruck vor.

Der Verfahrensschritt (a) kann in Gegenwart eines basischen Katalysators durchgeführt werden. Insbesondere bei der Reaktionsführung mit einem Trifluoressigsäureester ist der Zusatz einer Base besonders bevorzugt. Bei der Reaktionsführung mit Trifluoressigsäureanhydrid arbeitet man dagegen besonders bevorzugt ohne Base.

Als Basen dienen beispielsweise Alkalimetallhydroxide wie Natrium- und Kaliumhydroxid, Erdalkalimetallhydroxide wie Calcium- und Magnesiumhydroxid, Erdalkalimetalloxide wie Calcium- und Magnesiumoxid, Erdmetallhydroxide wie Aluminiumhydroxid, Alkalimetallcarbonate wie Natrium- und Kaliumcarbonat, Erdalkalimetallcarbonate wie Calcium- und Magnesiumcarbonat, Alkalimetallhydrogencarbonate wie Natrium- und Kaliumhydrogencarbonat, Erdalkalimetallhydrogencarbonate wie Magnesium- und Calciumhydrogencarbonat, Alkalimetallacetate wie Natrium- und Kaliumacetat, Erdalkalimetallacetate wie Magnesium- und Calciumacetat, Alkalimetall- oder Erdalkalimetallalkoholate wie Natrium- und Kaliummethanolat, Natrium- und Kaliumethanolat, Natrium- und Kalium-n-propanolat, Natrium- und Kaliumisopropanolat, Natrium- und Kalium-tert.-butanolat, Magnesiummethanolat und Magnesiumethanolat, tertiäre aliphatische Amine wie Trimethylamin, Triethylamin, Tri-n-propylamin, Triisopropylamin, Tri-n-butylamin, Dimethylethylamin, Dimethyldodecylamin und Dimethyl-tert.-butylamin, tertiäre cycloaliphatische Amine wie 1,8-Diazabicyclo[2.2.2]octan und 1,8-Diazabicyclo[5.4.0]undec-7-en, tertiäre Aniline wie N,N-Dimethylanilin, N,N-Diethylanilin, N-Ethyl-N-methylanilin und Methyldiphenylamin sowie tertiäre heterocyclische Amine wie 4-(Dimethylamino)pyridin.

Die Menge an Base beträgt üblicherweise 10 bis 200 mol-% der Menge des eingesetzten N-Trichloracetamidinoguanidins II. Größere Mengen sind möglich, bringen in der Regel aber keine weiteren Vorteile. Bei der Reaktionsführung mit einem Trifluoressigsäureester sind äquimolare Mengen an Base und N-Trichloracetamidinoguanidin II besonders bevorzugt.

In einer besonders bevorzugten Ausführungsform legt man das N-Trichloracetamidinoguanidin II in einem Lösungsmittel vor und dosiert den Trifluoressigsäureester III zu.

Der Verfahrensschritt (a) kann sowohl kontinuierlich als auch diskontinuierlich durchgeführt werden. Bei der kontinuierlichen Arbeitsweise leitet man die Reaktionspartner vorzugsweise durch einen Rohrreaktor.

Die Aufarbeitung des Reaktionsgemisches erfolgt allgemein in der Weise, daß man die niedrigsiedenden Bestandteile unter reduziertem Druck entfernt, Säurereste neutralisiert und die anorganischen Bestandteile durch Verrühren des Rohproduktes mit Wasser herauslöst.

Die Substitution der Trichlormethylgruppe des im Verfahrensschritt (a) erhaltenen 4-Trichlormethyl-6-trifluormethyl-1,3,5-triazins IV durch eine Alkoxygruppe erfolgt in einem inerten Lösungsmittel wie für den Verfahrensschritt (a) genannt, sofern sich nicht das Arbeiten in einem Überschuß des Alkohols V empfiehlt.

Zweckmäßigerweise führt man die Reaktion in Gegenwart eines basischen Katalysators durch. Als Basen kommen dabei Alkalimetallalkoholate sowie Amine wie oben genannt in Betracht. Besonders bevorzugt verwendet man die Alkalimetallalkoholate des jeweiligen Alkohols V.

Die Menge an Lösungsmittel ist nicht kritisch. Normalerweise verwendet man die 5- bis 10-fache Menge an Lösungsmittel, bezogen auf die Menge an 4-Trichlormethyl-6-trifluormethyl-1,3,5-triazin IV, sofern man nicht lösungsmittelfrei in einem Überschuß an Alkohol V arbeitet.

Die Menge an Alkohol oder Thiol der Formel V muß für eine vollständige Umsetzung mindestens äquimolar zur eingesetzten Menge an 4-Trichlormethyl-6-trifluormethyl-1,3,5-triazin IV sein. Arbeitet man ohne Lösungsmittel, so werden bevorzugt Mengen von 4 bis 5 Mol des Alkohols bzw. Thiols V, bezogen auf die Menge an 4-Trichlormethyl-6-trifluormethyl-1,3,5-triazin IV, verwendet.

Die Menge an Base beträgt im allgemeinen 1 bis 200 mol-%, bezogen auf die Menge an 4-Trichlormethyl-6-trifluormethyl-1,3,5-triazin IV. Bevorzugt verwendet man 5 bis 50 mol-% an Base bei $C_1$- und $C_2$-Alkoholen bzw. Thiolen V und über 50 mol-% bei Verbindungen V mit 3 und mehr Kohlenstoffatomen.

Vorzugsweise führt man bei Verfahrensschritt (b) bei Temperaturen zwischen 0°C und der Siedetemperatur des jeweiligen Lösungsmittels, bevorzugt zwischen 20 und 150°C, insbesondere zwischen 40 und 80°C durch.

Die Aufarbeitung des Reaktionsgemisches erfolgt wie üblich, und zwar in der Regel durch Entfernen der niedrigsiedenden Bestandteile bei reduziertem Druck nach Neutralisierung.

Der Verfahrensschritt (b) kann sowohl kontinuierlich als auch diskontinuierlich durchgeführt werden. Bei der kontinuierlichen Arbeitsweise leitet man die Reaktionspartner beispielsweise über ein Festbett aus einer unlöslichen Base oder man führt die Reaktion in einer mit dem Produkt I gesättigten Lösung unter laufender Entfernung von neu gebildetem Produkt durch.

Eine Variante des erfindungsgemäßen Verfahrens besteht darin, das Verfahrensprodukt IV des Schrittes (a) ohne Isolierung aus der Reaktionsmischung dem Verfahrensschritt (b) zu unterwerfen, wobei saure Nebenprodukte aus (a) durch eine höhere Konzentration an basischen Katalysator neutralisiert werden können. Diese Verfahrensweise kann dahingehend abgewandelt werden, daß man nach Durchführung des Schrittes (a) die niedrigsiedenden Bestandteile entfernt und das erhaltene Rohprodukt, gewünschtenfalls in einem anderen geeigneten Lösungsmittel, dem Verfahrensschritt (b) unterwirft.

Das erfindungsgemäßen Verfahren läßt sich mit Erfolg zur Synthese aller definitionsgemäßen 6-Trifluormethyl-1,3,5-triazine I anwenden, vor allem der in Tabelle 1 enthaltenen Verbindungen. Die 6-Trifluormethyl-1,3,5-triazine I sind wertvolle Zwischenprodukte für Pflanzenschutzmittel, wie sie beispielsweise aus der EP-A 111 442 und der DE-A 39 09 146 bekannt sind.

Tabelle 1

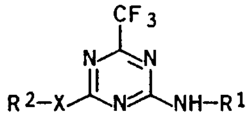

| Nr. | X | $R^1$ | $R^2$ | Fp[°C] |
|---|---|---|---|---|
| 1 | O | H | $CH_3$ | 163-165 |
| 2 | O | H | $C_2H_5$ | 124-128 |
| 3 | O | H | n-$C_3H_7$ | 100-103 |
| 4 | O | H | i-$C_3H_7$ | |
| 5 | O | H | n-$C_4H_9$ | 93- 94 |
| 6 | O | H | i-$C_4H_9$ | 102-104 |
| 7 | O | H | s-$C_4H_9$ | |
| 8 | O | H | t-$C_4H_9$ | |
| 9 | O | H | $CH_2CH=CH_2$ | 102-104 |
| 10 | O | H | E-$CH_2CH=CHCH_3$ | |
| 11 | O | H | $CH_2C≡CH$ | |
| 12 | O | H | $CH_2C≡CCH_3$ | |
| 13 | O | H | Cyclopropyl | |
| 14 | O | H | Cyclobutyl | |
| 15 | O | H | Cyclopentyl | |
| 16 | O | H | Cyclohexyl | |
| 17 | O | $CH_3$ | $CH_3$ | 134-135 |
| 18 | O | $CH_3$ | $C_2H_5$ | |
| 19 | O | $CH_3$ | n-$C_3H_7$ | |
| 20 | O | $CH_3$ | i-$C_3H_7$ | |
| 21 | O | $CH_3$ | n-$C_4H_9$ | |
| 22 | O | $CH_3$ | i-$C_4H_9$ | |
| 23 | O | $CH_3$ | s-$C_4H_9$ | |
| 24 | O | $CH_3$ | t-$C_4H_9$ | |
| 25 | O | $CH_3$ | $CH_2CH=CH_2$ | |
| 26 | O | $CH_3$ | E-$CH_2CH=CHCH_3$ | |
| 27 | O | $CH_3$ | $CH_2C≡CH$ | |
| 28 | O | $CH_3$ | $CH_2C≡CCH_3$ | |
| 29 | O | $CH_3$ | Cyclopropyl | |
| 30 | O | $CH_3$ | Cyclobutyl | |
| 31 | O | $CH_3$ | Cyclopentyl | |
| 32 | O | $CH_3$ | Cyclohexyl | |
| 33 | O | $C_2H_5$ | $CH_3$ | |
| 34 | O | $C_2H_5$ | $C_2H_5$ | |
| 35 | O | $C_2H_5$ | n-$C_3H_7$ | |

Fortsetzung Tabelle 1

| Nr. | X | $R^1$ | $R^2$ |
|---|---|---|---|
| 36 | O | $C_2H_5$ | $i-C_3H_7$ |
| 37 | O | $C_2H_5$ | $n-C_4H_9$ |
| 38 | O | $C_2H_5$ | $i-C_4H_9$ |
| 39 | O | $C_2H_5$ | $s-C_4H_9$ |
| 40 | O | $C_2H_5$ | $t-C_4H_9$ |
| 41 | O | $C_2H_5$ | $CH_2CH=CH_2$ |
| 42 | O | $C_2H_5$ | $E-CH_2CH=CHCH_3$ |
| 43 | O | $C_2H_5$ | $CH_2C\equiv CH$ |
| 44 | O | $C_2H_5$ | $CH_2C\equiv CCH_3$ |
| 45 | O | $C_2H_5$ | Cyclopropyl |
| 46 | O | $C_2H_5$ | Cyclobutyl |
| 47 | O | $C_2H_5$ | Cyclopentyl |
| 48 | O | $C_2H_5$ | Cyclohexyl |
| 49 | O | $n-C_3H_7$ | $CH_3$ |
| 50 | O | $i-C_4H_9$ | $C_2H_5$ |
| 51 | O | $n-C_3H_7$ | $n-C_3H_7$ |
| 52 | O | $i-C_4H_9$ | $i-C_3H_7$ |
| 53 | O | $n-C_3H_7$ | $n-C_4H_9$ |
| 54 | O | $s-C_4H_9$ | $i-C_4H_9$ |
| 55 | O | $i-C_3H_7$ | $s-C_4H_9$ |
| 56 | O | $t-C_4H_9$ | $t-C_4H_9$ |
| 57 | O | $i-C_3H_7$ | $CH_2CH=CH_2$ |
| 58 | O | $t-C_4H_9$ | $E-CH_2CH=CHCH_3$ |
| 59 | O | $i-C_3H_7$ | $CH_2C\equiv CH$ |
| 60 | O | $s-C_4H_9$ | $CH_2C\equiv CCH_3$ |
| 61 | O | $i-C_3H_7$ | Cyclopropyl |
| 62 | O | $i-C_4H_9$ | Cyclobutyl |
| 63 | O | $i-C_3H_7$ | Cyclopentyl |
| 64 | O | $t-C_4H_9$ | Cyclohexyl |

Herstellungsbeispiele

Beispiel 1

2-Amino-4-trichlormethyl-6-trifluormethyl-1,3,5-triazin

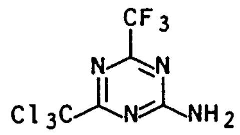

Verfahrensvariante 1 (ohne Base)

Unter starker Kühlung wurden zu einer Lösung aus 320 g (1,52 mol) Trifluoracetanhydrid und 500 ml Diethylether unter kräftigem Rühren bei 0 °C in 3 Portionen 150 g (0,74 mol) N-Trichloracetamidinoguanidin gegeben, wobei die Zugabe der letzten Portion ohne Kühlung erfolgte. Man rührte noch 3 Stunden, entfernte die niedrig siedenden Anteile bei 50 °C unter reduziertem Druck und versetzte den Rückstand mit 1,5 l Methylenchlorid. Die organische Phase wurde mit 3-normaler Natronlauge neutral gewaschen und über Natriumsulfat getrocknet. Nach Entfernen des Lösungsmittels erhielt man ein kristallines Rohprodukt, das 2 Stunden in 200 ml Wasser suspendiert wurde. Danach trennte man den Rückstand ab und löste ihn in Methylenchlorid. Nach Trocknen der Lösung wurde das Lösungsmittel unter reduziertem Druck entfernt. Ausbeute: 85 %

Verfahrensvariante 2 (mit Base)

Es wurden jeweils 0,1 mol N-Trichloracetamidinoguanidin mit n mol Trifluoressigsäureethylester in Gegenwart von 0,1 mol einer Base B bei T °C für eine Reaktionszeit von t Stunden zur Reaktion gebracht. Die übliche Aufarbeitung lieferte das 2-Amino-4-trichlormethyl-6-trifluormethyl-1,3,5-triazin (I) in einer Ausbeute von Z %.

Die Einzelheiten dieser Versuche sind Tabelle 2 zu entnehmen:

Tabelle 2

| Versuch | n [mol] | Base B | T[°C] | t[h] | Z |
|---|---|---|---|---|---|
| 1 | 0,5 | 1,4-Diazabicyclo[2.2.2]octan | 62 | 0,7 | 83 |
| 2 | 0,5 | 4-Dimethylaminopyridin | 62 | 2 | 84 |
| 3 | 0,5 | 1,8-Diazabicyclo[5.4.0]undec-7-en | 62 | 16 | 81 |
| 4 | 0,45 | 30 %ige Lösung $CH_3ONa$ in $CH_3OH$ | 0-5 | 0,2 | 95 |

Physikalische Daten:

Fp.: 112 bis 114 °C; $^1$H-NMR (in $(CD_3)_2SO$; 270 MHz, TMS als Standard [ppm]): 9,07 (s,br); 9,02 (s,br); $^{13}$C-NMR (in $(CD_3)_2SO$; 67,9 MHz, TMS als Standard [ppm]): 173,9 (s); 167,8 (s) ; 165,1 (q, $^2J_{C-F}$ = 37 Hz); 118,8 (q, $^1J_{C-F}$ = 277 Hz); 95,4 (s).

Beispiel 2

2-Amino-4-methoxy-6-trifluormethyl-1,3,5-triazin

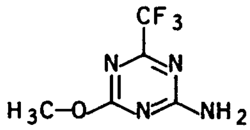

Zu einer Lösung von 28,8 g (0,1 mol) 2-Amino-4-trichlormethyl-6-trifluormethyl-1,3,5-triazin in 100 ml Methanol wurden bei 22°C 1,8 g einer 30 gew.-%igen Lösung von Natriummethanolat in Methanol (entsprechend 0,01 mol Natriummethanolat) gegeben. Die hellgelbe Lösung wurde anschließend 30 Minuten auf Rückflußtemperatur erhitzt. Das Fortschreiten der Reaktion wurde dabei mittels Dünnschichtchromatographie an Silikagel (Laufmittel: Diethylether/Hexan 1:1) verfolgt. Nach beendeter Reaktion versetzte man die Mischung mit 20 ml Wasser und dann mit 2-normaler Salzsäure, bis ein pH-Wert zwischen 6 und 7 erreicht war. Danach wurde der größte Teil des Methanols unter reduziertem Druck bei 40°C entfernt, wobei nach und nach 150 ml Wasser so zugegeben wurden, daß das Produkt nicht agglomerierte. Zur vollständigen Abscheidung des Produktes rührte man das erhaltene Gemisch noch 1 Stunde bei 5°C, wonach das Produkt abgetrennt, mit wenig Wasser gewaschen und unter reduziertem Druck bei 40°C getrocknet wurde.

Ausbeute: 95 %; Fp.: 163 bis 165°C; $^1$H-NMR (in $(CD_3)_2SO$; 270 MHz, TMS als Standard [ppm]): 8,35 (br); 8,17 (br); 8,17 (br); 3,99 (s);
$^{13}$C-NMR (in $(CD_3)_2SO$; 7,9 MHz, TMS als Standard [ppm]): 171,7 (s); 168,9 (s); 165,2 (q, $^2J_{C-F}$ = 36 Hz); 119,1 (q, $^1J_{C-F}$ = 277 Hz); 55,1 (s).

Beispiel 3

2-Amino-4-ethoxy-6-trifluormethyl-1,3,5-triazin

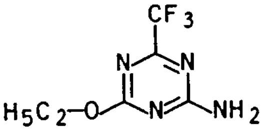

Zu einer Lösung von 0,48 g (7,1 mmol) Natriumethanolat in 100 ml Ethanol wurden bei 22°C 20,0 g (71 mmol) 2-Amino-4-trichlormethyl-6-trifluormethyl-1,3,5-triazin gegeben. Die hellgelbe bis hellgrüne Lösung wurde anschließend 30 Minuten auf Rückflußtemperatur erhitzt, nach Abkühlung auf 20°C mit 20 ml Wasser versetzt und mit 2-normaler Salzsäure neutralisiert. Danach wurde der größte Teil des Lösungsmittels unter reduziertem Druck bei 40°C entfernt, wobei nach und nach 100 ml Wasser so zugegeben wurden, daß die festen Anteile nicht zusammenklumpten. Das Produkt wurde analog Beispiel 2 isoliert.

Ausbeute: 92 %; Fp.: 124 bis 128°C; $^1$H-NMR (in $(CD_3)_2SO$; 270 MHz, TMS als Standard [ppm]): 8,26 (br); 8,14 (br); 4,42 (q; $^3J_{H-H}$ = 7 Hz); 1,37 (t; $^3J_{H-H}$ = 7 Hz);
$^{13}$C-NMR (in $(CD_3)_2SO$; 7,9 MHz, TMS als Standard [ppm]): 171,6; 169,0; (q, $^2J_{C-F}$ = 38 Hz); 118,8 (q, $^1J_{C-F}$ = 277 Hz); 64,7; 14,2.

Beispiel 4

2-Amino-4-(n-propoxy)-6-trifluormethyl-1,3,5-triazin

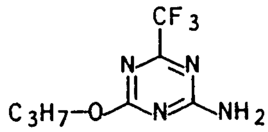

20,0 g (71 mmol) 2-Amino-4-trichlormethyl-6-trifluormethyl-1,3,5-triazin wurden bei 22 °C zu einer Lösung von 0,58 g (7,1 mmol) Natriumpropanolat in 40 ml Propanol gegeben. Anschließend erhitzte man die entstandene gelbe Mischung 30 Minuten auf 75 °C, versetzte sie dann mit weiteren 0,58 g (7,1 mmol) Natriumpropanolat in 10 ml Propanol und erhitzte noch 10 Minuten auf 75 °C. Nach dem Abkühlen auf 20 °C wurde die Reaktionsmischung mit 2-normaler wäßriger Salzsäure neutralisiert. Danach entfernte man unter reduziertem Druck die Hauptmenge des Lösungsmittels und verdünnte den Rückstand langsam mit 40 ml Wasser. Der gebildete Feststoff wurde abgetrennt, mit 5 ml Wasser gewaschen und unter reduziertem Druck bei 40 °C getrocknet.

Ausbeute: 93 %; Fp.: 100 bis 103 °C; $^1$H-NMR (in $CDCl_3$; 270 MHz, TMS als Standard [ppm]): 6,95 (br); 6,02 (br); 4,34 (t; $^3J_{H-H}$ = 7 Hz); 1,82 (sext; $^3J_{H-H}$ = 7 Hz), 1,03 (t; $^3J_{H-H}$ = 7 Hz);
$^{13}$C-NMR (in $CDCl_3$; 68 MHz, TMS als Standard [ppm]): 171,9; 169,1; 166,5 (q; $^2J_{C-F}$ = 37 Hz); 118,9 (q; $^1J_{C-F}$ = 277 Hz); 70,4; 22,1; 10,3.

Beispiel 5

2-Amino-4-methoxy-6-trifluormethyl-1,3,5-triazin

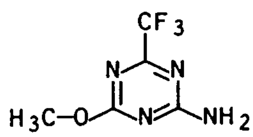

2,7 g (18 mmol) 1,8-Diazabicyclo[5.4.0]undec-7-en wurden bei 22 °C zu einer Lösung von 5 g (18 mmol) 2-Amino-4-trichlormethyl-6-trifluormethyl-1,3,5-triazin in 25 ml Methanol gegeben. Die Mischung wurde 1 Stunde auf Rückflußtemperatur erhitzt und nach dem Abkühlen auf 20 °C mit 2-normaler wäßriger Salzsäure schwach angesäuert (pH = 6). Anschließend entfernte man unter reduziertem Druck die Hauptmenge des Lösungsmittels, wonach man den gebildeten Feststoff wie üblich isolierte.

Ausbeute: 89 %; Fp.: 163 bis 165 °C

Beispiel 6

2-Methoxy-4-methylamino-6-trifluormethyl-1,3,5-triazin

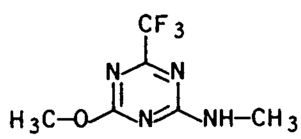

21,8 g einer 30 %igen Lösung von Natriummethanolat in Methanol (0,12 mol Natriummethanolat) wurden zu einer Lösung von 119,4 g (0,40 mol) 2-Methylamino-4-trichlormethyl-6-trifluormethyl-1,3,5-triazin in 400 ml Methanol getropft. Anschließend erhitzte man die entstandene gelbe Mischung 1 Stunde auf Rückflußtemperatur, wobei allmähliche Trübung beobachtet wurde. Nach Zugabe von weiteren 7,2 g der 30 %igen

Lösung aus Natriummethanolat in Methanol (0,04 mol Natriummethanolat) wurde die Mischung noch 30 Minuten auf Rückflußtemperatur erhitzt, nach dem Abkühlen auf 20 bis 25 °C mit 150 ml Wasser verdünnt und mit 1-normaler wäßriger Salzsäure neutralisiert. Anschließend entfernte man das Lösungsmittel unter reduziertem Druck bei 40 °C, wonach der entstandene Feststoff abgetrennt und 15 Stunden unter reduziertem Druck bei 40 °C getrocknet wurde.

Ausbeute: 93 %; Fp.: 134 bis 135 °C (Rotamerengemisch);
$^1$H-NMR (in $CDCl_3$; 270 MHz, TMS als Standard [ppm]): 6,51 (br); 3,20 (d) und 6,28 (br); 3,16 (d);
$^{13}$C-NMR (in $CDCl_3$; 67,9 MHz, TMS als Standard [ppm]): 175,2 (s); 167,1 (s); 165,7 (q, $^2J_{C-F}$ = 39 Hz); 118,5 (q, $^1J_{C-F}$ = 278 Hz); 95,2 (s) und 174,2 (s); 167,1 (s); 166,0 (q; $^2J_{C-F}$ = 39 Hz); 118,5 (q; $^1J_{C-F}$ = 278 Hz); 95,2 (s).

Beispiel 7

Einstufige Synthese von 2-Amino-4-methoxy-6-trifluormethyl-1,3,5-triazin (vgl. Beispiel 5)

Zu einer Lösung von 20,0 g (98 mmol) N-(Trichloracetamidino)guanidin in 100 ml Methanol tropfte man unter Rühren bei 0 °C eine Lösung von 25,3 g (197 mmol) Trifluoressigsäuremethylester in 20 ml Methanol und anschließend bei 0 bis 5 °C eine Lösung von 10,6 g (197 mmol) Natriummethanolat in 70 ml Methanol. Nach weiteren 5 Minuten Rühren bei 0 bis 10 °C konnte mittels Dünnschichtchromatographie [an Silikagel; Laufmittel: Diethylether/Hexan 1:1] die quantitative Bildung von 2-Amino-4-trichlormethyl-6-trifluormethyl-1,3,5-triazin nachgewiesen werden. Die Reaktionsmischung wurde nun solange bei 50 °C gerührt, bis dieses Zwischenprodukt nicht mehr nachzuweisen war (ca. 30 Minuten), dann auf 10 °C gekühlt und mit 50 ml Wasser versetzt. Man neutralisierte mit 10 gew.-%iger wäßriger Salzsäure (pH-Wert = 6 bis 7) und entfernte das Methanol unter reduziertem Druck bei 40 °C, wobei 50 ml Wasser so zugegeben wurden, daß das Rohprodukt nicht verklumpte. Zur vollständigen Abscheidung des Produktes rührte man noch 1 Stunde bei 5 °C, wonach es abgetrennt, mit wenig Wasser gewaschen und unter reduziertem Druck bei 40 °C getrocknet wurde. Ausbeute: 83 %; Fp.: 163-165 °C.

## Patentansprüche

1. Verfahren zur Herstellung von 6-Trifluormethyl-1,3,5-triazinen der allgemeinen Formel I

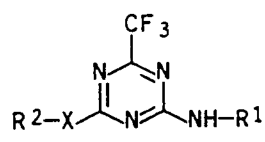

I

in der X Sauerstoff, $R^1$, $R^2$ C-organische Substituenten mit 1 bis 6 C-Atomen und $R^1$ zusätzlich Wasserstoff bedeuten, dadurch gekennzeichnet, daß man

(a) ein N-Trichloracetamidinoguanidin der allgemeinen Formel II

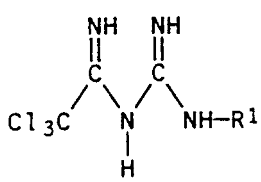

II

mit einem Trifluoressigsäurederivat der allgemeinen Formel III

$CF_3$-CO-Y III

in der Y Chlor, $C_1$-$C_4$-Alkoxy oder Trifluoracetyloxy bedeutet, in Gegenwart oder Abwesenheit einer Base zu einem 4-Trichlormethyl-6-trifluormethyl-1,3,5-triazin der Formel IV

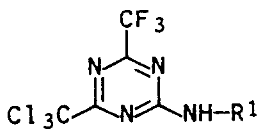

IV

umsetzt und

(b) das Verfahrensprodukt IV in Gegenwart einer Base mit einem Alkohol der allgemeinen Formel V

$R^2$-OH V

in der $R^2$ einen C-organischen Substituenten mit 1 bis 6 C-Atomen darstellt,

reagieren läßt.

2. Verfahren nach Anspruch 1, dadurch gekennzeichnet, daß man ein Trifluoressigsäurederivat III verwendet, wobei Y eine $C_1$-$C_4$-Alkoxygruppe bedeutet.

3. Verfahren nach Anspruch 1, dadurch gekennzeichnet, daß man ein Trifluoressigsäurederivat III verwendet, wobei Y die Trifluoracetyloxygruppe bedeutet.

4. Verfahren nach Anspruch 1, dadurch gekennzeichnet, daß man den Verfahrensschritt (a) in Gegenwart eines basischen Katalysators vornimmt.

5. Verfahren nach Anspruch 1, dadurch gekennzeichnet, daß man es auf die Herstellung von Verbindungen I anwendet, in denen $R^1$ Wasserstoff oder eine $C_1$-$C_4$-Alkylgruppe bedeutet.

6. Verfahren nach Anspruch 1, dadurch gekennzeichnet, daß man es auf die Herstellung von Verbindungen I anwendet, in denen $R^2$ einen $C_1$-$C_4$-Alkyl-, $C_3$-$C_4$-Alkenyl-, $C_3$-$C_4$-Alkinyl- oder $C_3$-$C_6$-Cycloalkylrest bedeutet.

7. Verfahren nach Anspruch 1, dadurch gekennzeichnet, daß man das 4-Trichlormethyl-6-trifluormethyl-1,3,5-triazin IV ohne Isolierung aus der Reaktionsmischung dem Verfahrensschritt (b) unterwirft.

## Claims

1. A process for preparing a 6-trifluoromethyl-1,3,5-triazine of the formula I

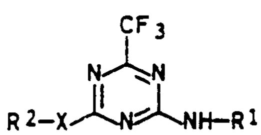

I

where X is oxygen, $R^1$ and $R^2$ are each carbon organic substituents with 1 to 6 carbons and $R^1$ is additionally hydrogen, which comprises

(a) reacting an N-trichloroacetamidinoguanidine of the formula II

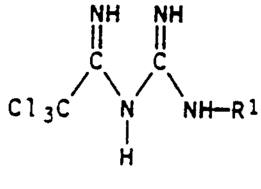

II

with a trifluoroacetic acid derivative of the formula III

$CF_3$-CO-Y III

where Y is chlorine, $C_1$-$C_4$-alkoxy or trifluoroacetoxy, in the presence or absence of a base to give a 4-trichloromethyl-6-trifluoromethyl-1,3,5-triazine of the formula IV

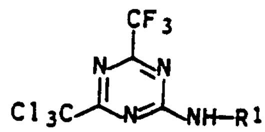

IV

and

(b) reacting the product IV in the presence of a base with an alcohol of the formula V

$R^2$-OH V

where $R^2$ is a carbon organic substituent with 1 to 6 carbons.

2. A process as claimed in claim 1, wherein a trifluoroacetic acid derivative III where Y is $C_1$-$C_4$-alkoxy is used.

3. A process as claimed in claim 1, wherein a trifluoroacetic acid derivative III where Y is trifluoroacetoxy is used.

4. A process as claimed in claim 1, wherein step (a) is carried out in the presence of a basic catalyst.

5. A process as claimed in claim 1, which is used to prepare a compound I where $R^1$ is hydrogen or $C_1$-$C_4$-alkyl.

6. A process as claimed in claim 1, which is used to prepare a compound I where $R^2$ is $C_1$-$C_4$-alkyl, $C_3$-$C_4$-alkenyl, $C_3$-$C_4$-alkynyl or $C_3$-$C_6$-cycloalkyl.

7. A process as claimed in claim 1, wherein the 4-trichloromethyl-6-trifluoromethyl-1,3,5-triazine IV is not isolated from the reaction mixture before undergoing step (b).

**Revendications**

1. Procédé de préparation de 6-trifluorométhyl-1,3,5-triazine de la formule générale I

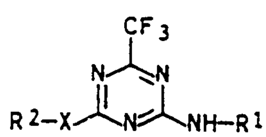

I

dans laquelle X représente l'oxygène, $R^1$, $R^2$ des substituants organiques en C à 1 à 6 atomes C et $R^1$ hydrogène supplémentaire, caractérisé par le fait que l'on fait réagir

(a) un N-trichloracétamidinoguanidine de la formule générale II

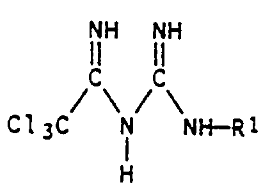

II

avec un dérivé d'acide trifluoroacétique de la formule générale III

$CF_3$-CO-Y III

dans laquelle Y représente chlore, alcoxy en C1-C4 ou trifluoracétyloxy, en présence ou absence d'une base, pour obtenir une 4-trichlorométhyl-6-trifluorométhyl-1,3,5-triazine de formule IV

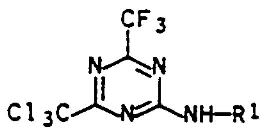

IV

et

(b) on fait réagir le produit IV du processus, en présence d'une base, avec un alcool de la formule générale V

$R^2$-OH V

dans laquelle $R^2$ représente un substituant organique en C avec 1 à 6 atomes C.

**2.** Procédé selon la revendication 1, caractérisé par le fait que l'on utilise un dérivé d'acide trifluoracétique III, Y signifiant un groupe alcoxy en C1-C4.

**3.** Procédé selon la revendication 1, caractérisé par le fait que l'on utilise un dérivé d'acide trifluoracétique III, Y signifiant le groupe trifluoracétyloxy.

**4.** Procédé selon la revendication 1, caractérisé par le fait que l'on effectue la phase (a) du procédé en présence d'un catalyseur basique.

**5.** Procédé selon la revendication 1, caractérisé par le fait qu'il est appliqué à la préparation de composés I, dans lesquels $R^1$ représente hydrogène ou un groupe alkyle en C1-C4.

**6.** Procédé selon la revendication 1, caractérisé par le fait qu'il est appliqué à la préparation de composés I dans lesquels $R^2$ représente un reste alkyle en C1-C4, alcényle en C3-C4, alcynyle en C3-C4, ou cycloalkyle en C3-C6, caractérisé par le fait qu'on soumet la 4-trichlorométhyl-6-trifluorométhyl-1,3,5-triazine IV, sans l'isoler du mélange de réaction, à la phase (b) du procédé.

**7.** Procédé selon la revendication 1, caractérisé par le fait qu'on soumet la 4-trichlorométhyl-6-trifluorométhyl-1,3,5-triazine IV, sans l'isoler du mélange de réaction, à la phase (b) du procédé.